# EUROPEAN PATENT APPLICATION

(11) **EP 1 974 762 A1**
(43) Date of publication of application: **01.10.2008**
(21) Application number: 07105164.3
(22) Date of filing: 29.03.2007
(51) Int. Cl.: A61M 16/00

(54) **Anaesthesia machine arrangement and a method in connection with an anaesthesia machine arrangement**

(71) Applicant: GENERAL ELECTRIC COMPANY, Schenectady, NY 12345 (US)
(72) Inventor: Tigerstedt, Erik, 02200, Espoo (FI)
(74) Representative: Valkeiskangas, Tapio Lassi Paavali

(57) **Abstract**

Anaesthesia machine arrangement and a method comprising a gas dispenser (10) of an anaesthesia machine connected to a patient circuit (13) comprising, a monitor device (7), a control device (9) and an interface unit (12). The gas dispenser (10) is configured to deliver a desired concentration of fresh gas (FG) to the patient circuit (13), the desired concentration being set by using the interface unit (12). The monitor device (7) is configured to monitor gas concentrations. The control device (9) is configured to control the gas dispenser (10) from the basis of the data received from the monitor device (7) to keep the desired fresh gas concentration. The monitor device (7) is configured to monitor and indicate if in the situation the fresh gas (FG) concentration is higher than expired gas (ET) concentration, the fresh gas concentration (FG) is lower that the expired gas (ET) concentration, or the fresh gas (FG) concentration is equal to the expired gas (ET) concentration, the inspired gas (Fi) concentration keeps changing.

## Description

The invention relates to an anaesthesia machine arrangement comprising a gas dispenser of an anaesthesia machine connected to a patient circuit comprising, a monitor device, a control device and an interface unit, whereby the gas dispenser is configured to deliver a desired concentration of fresh gas to the patient circuit, the desired concentration being set by using the interface unit, the monitor device is configured to monitor gas concentration in the patient circuit and the control device is configured to control the gas dispenser from the basis of the data received from the monitor device to keep the desired fresh gas concentration. The invention further comprises a method in connection with an anaesthesia machine arrangement.

A basic requirement set for devices used in patient care is that they are safe and operationally reliable in the normal use of the device, in fault situations unintentionally caused by a user or in any one-fault situation of the device.

Anaesthesia machines and ventilators used in intensive care and anaesthesia can be mentioned as examples of the devices described above. A patient is normally connected to a device used in patient care, e.g. an anaesthesia machine and ventilator, by means of a patient circuit. From the patient circuit there is a measuring connection to a monitor, which monitors the condition of the patient. Using measuring information on the condition of the patient a healthcare person supervises the condition of a patient and adjusts set values of the device used in patient care so that the measuring information corresponds to the desired value of the moment.

Due to the indirectness, slow and insufficient or excessive reactions from the user and in some circumstances long time constant the exact adjustment of measuring values is slow and difficult, which leads to variation in patient values, and this in turn may have harmful effects on the end result in nursing.

To improve the situation, a variety of solutions have been suggested for automatizing the systems used in the situations described above. In other words it has been suggested that a system, i.e. a machine and not a person, takes automatically care of adjusting steps needed to obtain a result desired. When discussing about automatic systems here we mean that automatic delivery of breathing gases to the patient refers to a system where the desired concentration in breathing gases is set and the machine used keeps the mixture correct by automatically adjusting the fresh gas (FG) flow concentration. When using automatic systems it is however essential that for example in an automatic system delivering a mixture of anaesthetic agent, N20 and oxygen to the patient to be treated, there must be some way to assure that a device failure does not cause the system to deliver wrong concentration to the patient.

In order to achieve a safe level high enough, double monitoring is used in the prior art. In double monitoring two or even more monitors are used so that a fault in one monitor can be brought to the user's attention if the monitors show different readings.

The disadvantages of this known technique is the high cost of double monitoring, i.e. double or in some cases even more than double monitoring hardware leads to high costs.

As another example of the prior art a system using one monitor and a sample system can be mentioned. In this system one monitor is used and samples are taken from either the fresh gas used or from some other known gas. The fresh gas concentration is known. If the fresh gas sample readings are wrong one knows that either the anaesthesia machine or monitor does not work correctly. This known system is described in EP Patent 1 140 264 B1.

The disadvantages of this known prior art system relate to gas sampling hardware, i.e. the hardware needed to take gas samples etc. leads to rather high costs.

The object of the invention is to provide an arrangement by which the prior art disadvantages can be eliminated. This is achieved by the arrangement according to the invention. The arrangement of the invention is characterized in that the monitor device is configured to monitor and indicate if in the situation where the fresh gas concentration is higher than expired gas concentration, the fresh gas concentration is lower than the expired gas concentration, or the fresh gas concentration is equal to the expired gas concentration, the inspired gas concentration keeps changing. The method of the invention is characterized by the step in which in the situation where the fresh gas concentration is higher than expired gas concentration, the fresh gas concentration is lower that the expired gas concentration, or the fresh gas concentration is equal to the expired gas concentration it is monitored and indicated if the inspired gas concentration keeps changing.

The advantage of the invention is that the high costs of the double monitoring and gas sampling hardware can be avoided. The invention can be implemented as a software solution only.

In the following the invention will be described in greater detail by means of the attached drawing in which
Figure 1 shows a schematic view of an operational environment of equipment used in patient care and
Figure 2 shows a diagram of an example of an automatic control arrangement of a gas dispenser in an anaesthesia machine.

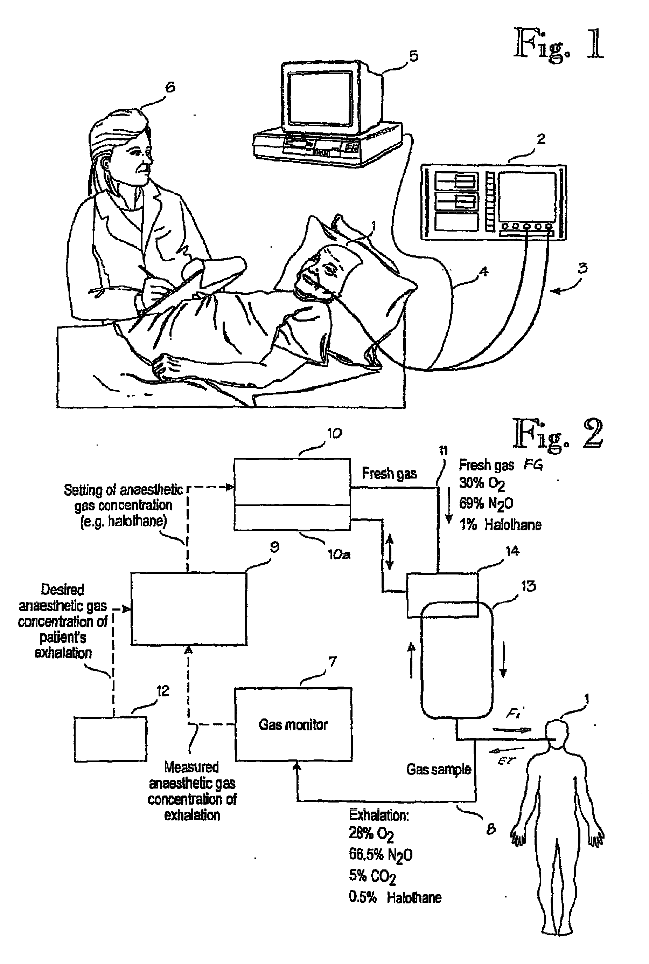

Figure 1 shows a schematic view of an operational environment of equipment used in patient care. A patient 1 is connected to a device used in patient care, which, in the example of Figure 1, is a combination of a gas mixer and ventilator 2. The patient is connected by means of a patient circuit 3. From the patient circuit 3 there is a measuring connection 4 to a monitor 5, which monitors the condition of the patient. A healthcare person 6 supervises the condition of the patient on the basis of the measuring information on the condition of the patient provided by the monitor 5 and, when necessary, adjusts set values of the device used in patient care such that the measuring information corresponds to the desired value of the moment, as described above.

Figure 2 shows an example of a control system, in which a gas dispenser of an anaesthesia machine is automatically controlled on the basis of a signal given by breathing gas measurement of the monitor. If, as a result of a fault situation, a gas monitor 7 measures a lower anaesthetic gas concentration 8 than it in fact is or does not measure it at all, a controller 9 sets a gas dispenser 10, to produce a higher anaesthetic gas concentration than it actually should be according to the value set by a user with an interface unit 12. This leads to an overdose of the anaesthetic and thus to a dangerous situation. Gas concentrations shown in Figure 2 are only example values. In Figure 2 a patient circuit is indicated with the reference number 13, an eventual CO2 absorber with the reference number 14 and a ventilator with the reference number 10a. As in the example of Figure 1 a patient is marked with the reference number 1.

In principle, the system of Figure 2 operates in the following way. While a patient is under treatment, the gas dispenser 10 feeds a desired gas mixture to the patient 1 and the gas monitor 7 measures the anaesthetic gas concentration and informs the controller 9 of it. The controller 9 adjusts the setting of the anaesthetic gas concentration in order to achieve the desired end result. The user has naturally set a desired gas concentration of the patient's exhalation to the controller 9 by using the interface unit 12.

The system shown in Figure 2 has the disadvantages described above if for example the gas monitor 7 for some reason measures lower concentration than it actually is.

The arrangement shown in EP 1 140 264 B1 eliminates the disadvantages described above. The arrangement shown in EP 1 140 264 B1 leads however to relatively high costs because considerable amount of gas sampling hardware must be used.

The present invention creates a solution by which the disadvantages described above can be eliminated without excessive cost related to additional hardware discussed for example above in connection with EP 1 140 264 B1.

Referring to Figure 2 and the present invention the inspired gas (Fi) concentrations are made up of expired gas (ET) - CO2 + fresh gas (FG). Fi and ET concentrations are all measured for all gases. The gas dispenser 10 is configured to deliver a desired concentration of fresh gas (FG) to the patient circuit 13. The desired concentration is set by using the interface unit 12. The monitor device 7 is configured to monitor inspired gas (Fi), expired gas (ET) concentrations and CO2 concentrations in the patient circuit 13. The control device 9 is configured to control the gas dispenser 10 from the basis of the data received from the monitor device 7 to keep the desired fresh gas (FG) concentration.

The invention starts from the fact that if the fresh gas (FG) concentration is higher than ET concentration the Fi concentration starts to rise, and correspondingly if the FG concentration is lower than the ET concentration the Fi concentration starts dropping. In the situation where the FG concentration is equal to the ET concentration the Fi is stable.

As told above the anaesthesia machine receives measuring information from the monitor device, i.e. the anaesthesia machine is controlled from the basis of the data received from the monitor device. If the monitor however gives false information a dangerous situation may occur. If the anaesthesia machine receives false information i.e. if the anaesthesia machine from the basis of the information from the monitor device 7 believes that it gives equal concentrations as ET but Fi keeps changing it can from the facts described above according to the invention be said that there is an error in the anaesthesia machine, monitor device or tubing. In other words according to the invention it is essential that the monitor device 7 is configured to monitor and indicate if in the situation the fresh gas (FG) concentration is equal to the expired gas (ET) concentration, the inspired gas (Fi) concentration keeps changing to find out if there exist any malfunctions in the system.

The arrangement can be provided to give any appropriate alarm signal if such an indication is needed.

To check the calibration the anaesthesia machine arrangement can be arranged to give a little more or less than the ET concentration and to check the Fi response. If the response is in the wrong direction something is wrong. After this the same procedure can be done in the other direction, i.e. if first more and the checking of the Fi response then less and checking the Fi response, or first less and checking the Fi response and then more and checking the Fi response.

The absolute concentration cannot be detected but it is between the values. By knowing the flows in the patient circuit and the CO2 concentration the differences can be chosen so that "between" is so small that the accuracy is sufficient.

The embodiment of the invention described above is by no means intended to restrict the invention but the invention may be modified completely freely within the scope of the claims.

## Claims

1. Anaesthesia machine arrangement comprising a gas dispenser (10) of an anaesthesia machine connected to a patient circuit (13) comprising, a monitor device (7), a control device (9) and an interface unit (12), whereby the gas dispenser (10) is configured to deliver a desired concentration of fresh gas (FG) to the patient circuit (13), the desired concentration being set by using the interface unit (12), the monitor device (7) is configured to monitor gas concentration in the patient circuit and the control device (9) is configured to control the gas dispenser (10) from the basis of the data received from the monitor device (7) to keep the desired fresh gas concentration, **characterized in that** the monitor device (7) is configured to monitor and indicate if in the situation where the fresh gas (FG) concentration is higher than expired gas (ET) concentration, the fresh gas concentration (FG) is lower that the expired gas (ET) concentration, or the fresh gas (FG) concentration is equal to the expired gas (ET) concentration, the inspired gas (Fi) concentration keeps changing.

2. Anaesthesia machine arrangement according to claim 1, **characterized in that** the monitor device (7) is configured to rise the fresh gas (FG) concentration slightly over the expired gas concentration (ET) to check if the inspired gas concentration (Fi) response is in the wrong direction.

3. Anaesthesia machine arrangement according to claim 1, **characterized in that** the monitor device (7) is configured to lower the fresh gas (FG) concentration slightly under the expired gas concentration (ET) to check in the inspired gas concentration (Fi) response is in the wrong direction.

4. Anaesthesia machine arrangement according to claim 1, **characterized in that** the monitor device is configured to monitor inspired gas (Fi) concentrations or/and expired gas (ET) concentrations comprising of anaesthetic agent, CO2, O2, N2, N2O and H2O.

5. Anaesthesia machine arrangement according to claim 1, **characterized in that** the patient circuit further comprises a CO2 absorber (14).

6. Method in connection with an anaesthesia machine arrangement comprising a gas dispenser (10) of an anaesthesia machine connected to a patient circuit (13) comprising, a monitor device (7), a control device (9) and an interface unit (12), in which method a desired concentration of fresh gas (FG) is delivered to the patient circuit (13), the desired concentration is set, gas concentration in the patient circuit is monitored and the gas dispenser (10) is controlled from the basis of the data received from the gas concentration monitoring to keep the desired fresh gas concentration, **characterized by** the step in which in the situation where the fresh gas (FG) concentration is higher than expired gas (ET) concentration, the fresh gas concentration (FG) is lower that the expired gas (ET) concentration, or the fresh gas (FG) concentration is equal to the expired gas (ET) concentration it is monitored and indicated if the inspired gas (Fi) concentration keeps changing.

7. Method according to claim 6, **characterized in that** the fresh gas (FG) concentration is made to rise slightly over the expired gas concentration (ET) to check if the inspired gas concentration (Fi) response is in the wrong direction.

8. Method according to claim 6, **characterized in that** the fresh gas (FG) concentration is lowered slightly under the expired gas concentration (ET) to check in the inspired gas concentration (Fi) response is in the wrong direction.

9. Method according to claim 6, **characterized in that** inspired gas (Fi) concentrations or/and expired gas (ET) concentrations comprising of anaesthetic agent, CO2, O2, N2, N2O and H2O are monitored.

10. Method according to claim 6, **characterized in that** the patient circuit further comprises a CO2 absorber.
